# EUROPEAN PATENT SPECIFICATION

(11) **EP 2 741 693 B1**
(45) Date of publication and mention of the grant of the patent: **07.10.2015**
(21) Application number: 12748358.4
(22) Date of filing: 10.08.2012
(51) Int. Cl.: A61B 17/3207, A61B 17/00, A61B 17/32

(54) **SYSTEMS FOR REMOVAL OF ATHEROSCLEROTIC PLAQUE OR THROMBUS AT A TREATMENT SITE**
SYSTEME ZUR ENTFERNUNG VON ATHEROSKLEROTISCHEM PLAQUE ODER VON THROMBEN AN EINER BEHANDLUNGSSTELLE
SYSTÈMES D'ÉLIMINATION DE PLAQUE D'ATHÉROSCLÉROSE OU DE TROMBUS AU NIVEAU D'UN SITE DE TRAITEMENT

(30) Priority: 12.08.2011 US 201161523225 P; 09.08.2012 US 201213571307
(43) Date of publication of application: 18.06.2014
(73) Proprietor: W.L. Gore & Associates, Inc., Newark DE 19711 (US)
(72) Inventor: CULLY, Edward H., Flagstaff, AZ 86004 (US); DUNCAN, Jeffrey B., Flagstaff, AZ 86001 (US); LUBER, Kaylan M., Flagstaff, AZ 86004 (US); MONTGOMERY, William D., Flagstaff, AZ 86001 (US); SHAW, Edward E., Flagstaff, AR 86004 (US)
(74) Representative: Wilson, Gary
(86) International application number: PCT/US2012/050396
(87) International publication number: WO 2013/025531

(56) References cited:
- WO-A2-2010/013213
- DE-A1- 3 921 071
- JP-A- 11 018 835
- US-A- 5 782 848
- US-A1- 2007 213 753

## Description

### FIELD OF THE INVENTION

The present disclosure relates to systems for reducing unwanted flow or leakage around medical devices installed in a treatment region, and more specifically for filling voids where medical devices are installed in a body lumen. The present disclosure further relates to systems and devices for the removal of atherosclerotic plaque or thrombus protruding from or through a deployed medical device such as a vascular stent.

### BACKGROUND

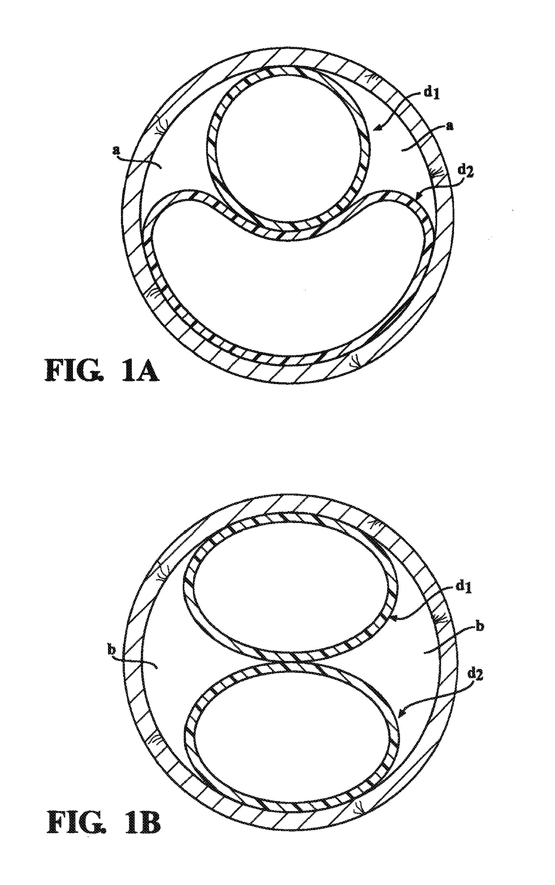

Treatment of various portions of the vasculature can require the installation of one or more medical devices. A medical device can be any device or structure configured to provide and/or support a therapeutic use in the vasculature. For example, stents or stent grafts, valves, bifurcated stents, and drug-delivering devices can be implanted in the vasculature at a treatment region. Typical medical devices can have geometries that do not conform to the vasculature. Moreover, during a medical procedure, a plurality of medical devices can be installed in a single region of the vasculature in what are sometimes referred to as "chimney," "snorkel," or "sandwich" arrangements, for example, as shown and generally indicated at "d1 : and "d2" in Figures 1A and 1 B. As a result, the cross section defined by the medical device(s) when installed in the vasculature can not equally correlate to the entire cross section of the vasculature where the medical device(s) is/are installed. Use of these typical medical devices in medical procedures therefore creates installations with "gutters" or "gutter regions," as illustrated at "a" and "b" also shown in Figures 1 A and 1 B. Flow into and/or through the gutters can be unwanted. For instance, unwanted flow can pressurize an aneurysm or create other problems with the vasculature in the treatment region due to persistent leaking or perfusion.

Thus, a need exists to reduce unwanted flow or leakage into or through gutters. As used herein, "leakage" means the unwanted or undesirable flow into or through a treatment region, where the flow is outside the lumen(s) or body(ies) defined by the medical device(s). Those skilled in the art will recognize numerous advantages of such exemplary embodiments over the prior art, including, for example, substantially reducing such flow in the gutter region with an implantable leakage reduction system.

DE3921071 (A1) is the closest prior art to the subject matter of claim 1 and discloses a method wherein a thrombosis (6) is removed by means of an instrument in the form of a semi-rigid rod (3) to which bristles (7,8) are attached so that they project radially from the rod. The blood vessel (1), in which the thrombosis occurs, is punctured at a point near the thrombosis. The instrument is inserted into the blood vessel so that it passes through the thrombosis. The instrument is moved backwards and forwards and at the same time is rotated so that the fibrinous substance forming the thrombosis, adheres to the bristles. The instrument is then withdrawn with the fibrinous substance adhering to the bristles.

US5782848 (A) relates to quickly and efficiently removing coagulated tissue within a body lumen without affecting the normal, healthy, untreated tissue, to minimize post-operative complications such as bleeding or edema formation. For example, in an aspect, the disclosure features a surgical resecting brush assembly. The assembly includes an elongated catheter, sized and constructed for delivery through an extended body lumen to a site that is occluded with body matter that has been effected by heat treatment, and a brush formation near the distal end of the catheter. The brush formation includes a bristle structure of sufficient stiffness to resect and remove the effected bodily matter without substantially damaging adjacent healthy tissue when the brush formation is actuated relative to the occluded site.

US2007213753 (A1) discloses an assembly and method for removing occlusive material from a stent. The assembly includes an elongate catheter with an elongate flexible shaft disposed therethrough and a cleaning end disposed distally upon the shaft. The cleaning end is a cleaning brush and is configured to engage and dislodge the occlusive material.

### SUMMARY OF THE INVENTION

In an embodiment, a leakage reduction system may comprise one or more components configured to fill open spaces or "gutters" around medical devices installed in the vasculature. The leakage reduction system or plaque/thrombus reduction device comprises any suitable structure configured to fill a gap or volume, including for example, a support structure, a frame, gel, beads, alginate, a balloon, a brush, or any other suitable support structure. The system or device may also comprise a coating or secondary structure, including, for example, bags, occluder disks, balloons, foams, gels, and the like. Exemplary coatings may attach and/or laminate to the support structure, and/or to the medical device itself, to further reduce leakage into or through a gutter. The leakage reduction system or plaque reduction device may also comprise a deployment mechanism. The deployment mechanism may be any suitable structure configured to removably attach the support structure or secondary structure to a delivery device. For example, the deployment mechanism may include a collar, threads, a clip, snare, noose, or any other suitable structure.

In various embodiments, the leakage reduction system or plaque/thrombus reduction device may comprise one or more components that are conformable or otherwise have geometries configured to cover substantially all of the cross-sectional area of a vessel.

In various embodiments the leakage reduction system may be used independently to obstruct flow through a vessel.

In various embodiments the leakage reduction system may be used to occlude anatomical features such as left atrial appendage.

In various embodiments the leakage reduction system may be used to obstruct flow though a cardiac defect.

An embodiment can include a plaque reduction brush comprising at least one support adapted to allow the brush to be endoluminally deliverable via a delivery device to a treatment site; and a plurality of bristles extending from the at least one support and being arranged in a predetermined pattern along the at least one support, each of the plurality of bristles having a length, wherein one or more of the plurality of bristles has a bending portion along the length that is more easily bent than another portion of the length thereof, said bending portion facilitating bending of the one or more of the plurality of bristles between a compressed delivery configuration and an expanded use configuration.

In some embodiments the delivery device can be a guidewire.

Some embodiments can have a secondary structure.

Some embodiments can have a secondary structure that is a bag.

Embodiments can have a bag that substantially covers one or more of the plurality of bristles.

Embodiments can have bristles that at least partially protrude from the bag.

Embodiments can have a bag that comprises a fluoropolymer.

Further embodiments can have bristles formed from nitinol.

Further embodiments can have bristles incorporate a strain relief adjacent to the support.

Embodiments can have bristles that are laminated in a fluoropolymer.

Additional embodiments can have one or more of the plurality of bristles has a substantially uniform cross section along the length thereof, the bending portion being defined by a portion of the one or more of the plurality of bristles having a reduced cross section.

Embodiments can have a plurality of bristles that extend outwardly from a generally annular collar fixedly secured to the support, the reduced width portion being adjacent the collar.

Embodiments can have one or more of the plurality of bristles has a generally flattened cross section.

Further embodiments have a bending portion that is defined by a generally sinusoidal-shaped bend along a portion of the length of the one or more of the plurality of bristles.

Further embodiments have a bending portion that is defined by a change of flex modulus along the length of the bristle.

Additional embodiments can have a predetermined path from which the plurality of bristles protrude from the support extends generally helically and obliquely about the support.

Additional embodiments can have a plurality of bristles that are polymer.

Embodiments can have one or more of the plurality of bristles which has a substantially uniform durometer along the length thereof, the bending portion having a reduced durometer.

Some embodiments can have one or more of the plurality of bristles which are covered such as with a hydrogel.

Embodiments can have a plurality of bristles covered with a PTFE tape wrapping.

Embodiments can have a tape wrapping which has a varying slit width.

Embodiments can have a tape wrapping that has a varying wrap angle.

Further embodiments can have one or more of plurality of bristles covered with a fluoropolymer.

### BRIEF DESCRIPTION OF THE DRAWINGS

The subject matter of the present disclosure is particularly pointed out and distinctly claimed. A more complete understanding of the present disclosure, however, can best be obtained by referring to the detailed description and claims when considered in connection with the drawing figures, wherein like numerals denote like elements and wherein:
Figures 1A-1 B illustrate cross sectional views of a section of human vasculature showing the gutter region defined by two medical devices;
Figures 2 illustrates an exemplary leakage reduction system or plaque/thrombus reduction device comprising a frame with cover;
Figures 3A-3B illustrate an exemplary leakage reduction system or plaque/thrombus reduction device comprising a filler;
Figure 4 illustrates an exemplary leakage reduction system or plaque/thrombus reduction device comprising a balloon;
Figure 5 illustrates an exemplary leakage reduction system or plaque/thrombus reduction device comprising a brush and a balloon;
Figures 6A-6E illustrate an exemplary leakage reduction system or plaque/thrombus reduction device comprising a brush;
Figures 7A-7D illustrate an exemplary leakage reduction system or plaque/thrombus reduction device comprising a brush and an occluder;
Figures 8A-8C illustrate an exemplary leakage reduction system or plaque/thrombus reduction device comprising a laminated brush;
Figure 9A-9B illustrate an exemplary leakage reduction system or plaque/thrombus reduction device comprising a brush and foam;
Figures 10A-10C illustrate an exemplary leakage reduction system or plaque/thrombus reduction device comprising a bag and brush; and
Figures 11A-11C illustrate an exemplary plaque removal or reduction system comprising a plaque removal or reduction device in combination with distal or proximal embolic protection devices; and
Figure 12 illustrates an exemplary plaque reduction system in the vasculature.
Figure 13 illustrates an exemplary plaque reduction device comprising a brush with bristles having bending portions.
Figure 14 illustrates an exemplary plaque reduction device comprising a brush with bristles having bending portions.

### DETAILED DESCRIPTION

The detailed description of various embodiments herein makes reference to the accompanying drawing figures, which show various embodiments and implementations thereof by way of illustration and best mode, and not of limitation. While these embodiments are described in sufficient detail to enable those skilled in the art to practice the embodiments, it should be understood that other embodiments can be realized and that mechanical and other changes can be made without departing from the scope of the present disclosure.
Furthermore, any reference to singular includes plural embodiments, and any reference to more than one component can include a singular embodiment. Moreover, recitation of multiple embodiments having stated features is not intended to exclude other embodiments having additional features or other embodiments incorporating different combinations of the stated features. In describing various embodiments, the term distal is used to denote the end of an exemplary device nearest to the treatment region within a patient's body. The term proximal is used to denote the end of an exemplary device nearest to the user or operator of the device.

The present disclosure relates to a number of non-limiting, exemplary embodiments, each of which can be used alone or in coordination with one another. A leakage reduction system can be any suitable system installable within the vasculature and configured to reduce leakage of blood and other bodily fluids during and/or after a medical procedure. A plaque/thrombus reduction device can be any suitable system for use within the vasculature and configured to reduce plaque and or thrombus from the vasculature and or deployed medical device (medical device that is in the vasculature). The leakage reduction system can comprise one or more components configured to fill open spaces or "gutters" around medical devices installed in the vasculature. The leakage reduction system or plaque/thrombus reduction device can comprise one or more components with geometries configured to cover substantially all of the cross- sectional area of a vessel. The leakage reduction system encourages flow through a lumen defined by installed medical devices and/or reduces the flow of blood and/or bodily fluids around the medical devices.
Exemplary leakage reduction systems described herein can provide benefits, such as modularity, the ability to use standard stent graft tubular combinations, reduced side branch complexity, antegrade or retrograde perfusion of side branches, revisions of leaks, not to mention it being a relatively simple technique.

The leakage reduction system or plaque/thrombus reduction device can comprise any suitable structure configured to fill a gap or to reduce plaque and or thrombus from the vasculature and or a medical device in the vasculature. The leakage reduction system may comprise a support structure, including for example, a frame, gel, beads, alginate, a balloon, a brush, or any other suitable support structure. In general, any structure that provides volume and/or increased surface area (e.g., for coagulation) to a gap can be used.

The leakage reduction system or plaque/thrombus reduction device can also comprise a coating or secondary structure, including, for example, a bag, an occluder disk, a balloon, foams, gels, and the like. Exemplary coatings can attach and/or laminate to the support structure, and/or the medical device itself, to further reduce leakage into or through a gutter or enhance plaque or thrombus reduction (removal of plaque or thrombus in whole or in part) from the treatment area.

In accordance with yet other examples, a support structure or secondary structure can comprise an elongate element modified to promote a desired therapeutic response like thrombogenesis, assist in increased hemostatic properties and/or enhance its delivery or deployment by, for example, adjusting a dimensional characteristic. The elongate element can be imbibed with a first reagent that serves to activate or react with a second reagent. For example, an elongate element can comprise alginic acid (aka alginate) cross-linked with one or more polyvalent compounds.

The leakage reduction system and plaque/thrombus reduction device can also comprise a radiopaque marker and a deployment mechanism. The radiopaque marker can allow a user to observe the position of the leakage reduction system in the body to properly position the system in a gutter or orient the device within a deployed stent containing protruding atherosclerotic plaque material or intraluminal thrombus. The deployment mechanism can be any suitable structure configured to removably attach the support structure or secondary structure to a delivery device. For example, the deployment mechanism can include a collar, threads, a clip, noose, snare, or any other suitable structure.

The leakage reduction system or plaque/thrombus reduction device can deploy endoluminally through any suitable medical device delivery system. The medical device delivery system can comprise a deployment mechanism, catheter, guidewire, or other suitable conduit for delivering the leakage reduction system to a treatment region. In these embodiments, the catheter, guidewire, or conduit can comprise a lumen configured to receive inputs and/or materials from the proximal end of the medical device delivery system and conduct the inputs and/or materials to the leakage reduction system at the treatment region.

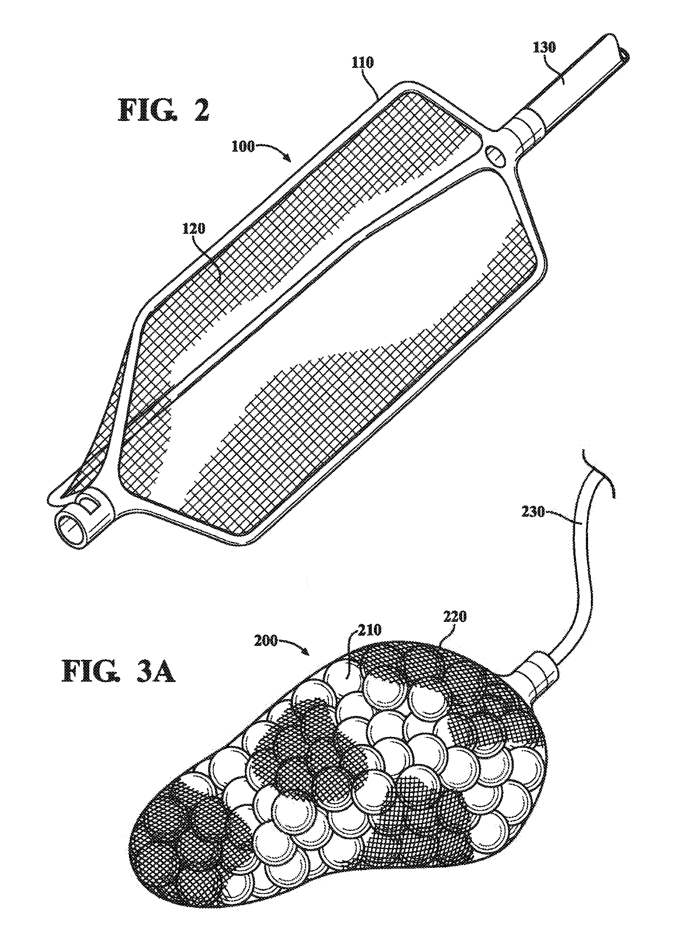

With reference to Figure 2, a leakage reduction system or plaque/thrombus reduction device 100 can comprise a frame 110, a bag 120, and a deployment mechanism 130. Frame 110 can be of any suitable size and shape to fill a gutter or remove in whole or in part plaque or thrombus. Frame 1 10 can support and couple to bag 120. Frame 1 10 can also comprise or otherwise couple to deployment mechanism 130. Frame 110 can be formed of laser cut tube, nitinol wire, or any other suitable biocompatible material. Bag 120 can be formed from ePTFE or any other suitable biocompatible material.

Bag 120 can couple to frame 110 such that frame 120 defines an open side. The open side can be installable around the medical device structure or vascular structure defining the gutter. Leakage reduction system 100 can also be oriented in the gutter to allow blood and/or bodily fluids to flow into the open side. When coupled to frame 110, bag 120 can be configured as an occluder bag. In various embodiments, the bag 120 can be porous so as to allow some blood and/or bodily fluid to flow therethrough when installed in the gutter. Over time, this flow can be substantially limited due to reduced porosity of bag 120 as a result of clotting, tissue in-growth, cross-linking, etc.

In use, the leakage reduction system or plaque/thrombus reduction device 100 can be delivered endoluminally to a treatment region through any suitable medical device delivery system. Such a delivery system can include, for example, a catheter or other suitable sheath. The delivery system can also include a deployment mechanism that releasably compresses the leakage reduction system or plaque/thrombus reduction device 100 to a compressed size or state suitable for for endoluminal delivery of the system to the treatment region. The system or device can then be expanded by releasing the deployment mechanism at the treatment region such as within the gutter or medical device. Deployment mechanisms can include constraining sleeves, sheaths, lines or tethers, or other structures suitable for releasably compressing the system to a size suitable for endoluminal delivery to the treatment region. This process can be repeated for installations with multiple gutters.

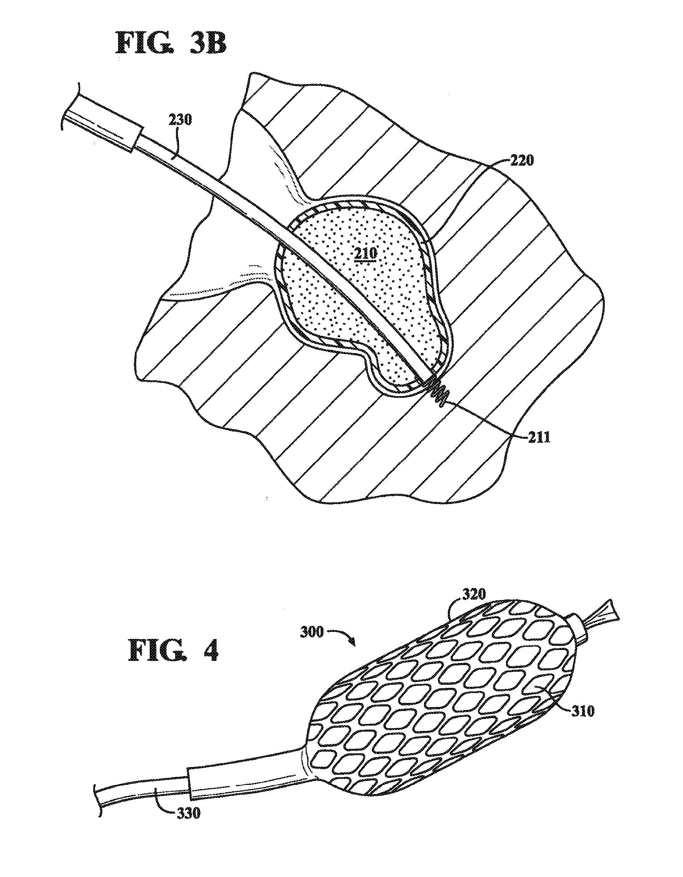

With reference to Figures 3A-3B, a leakage reduction system or plaque/thrombus reduction device 200 can comprise a filler 210, a bag 220, and a deployment mechanism 230. Leakage reduction system 200 can also comprise an attachment mechanism 211. The attachment mechanism can be any suitable structure configured to couple and/or engage the vasculature. For example, attachment mechanism 211 can include a coil, a hook, a barb, an anchor, or any other suitable structure capable of coupling to and/or engaging a vascular wall. Bag 220 can contain and/or house filler 210 and couple to and/or define deployment mechanism 230. Filler 210 can be any suitable substance, including, for example, a gel, a foam, beads, alginate, a multi-part substance, and/or the like. Bag 220 can be made of any suitable material, such as, for example, ePTFE or any other suitable material.

In one example, filler 210 is a gel, foam, or multi-part material. In this embodiment, bag 220 can deploy endoluminally through a medical device delivery system on a catheter to a treatment region. Bag 220 can be empty or can comprise an initial part of a multi-part substance. Bag 220 can be positioned in the gutter and filled. Attachment mechanism 211 can further couple bag 200 to a vascular wall to stabilize bag 220 in the gutter. As noted above, the deployment can a have a lumen configured to receive an input. In this example, the input can be one or more of gel, foam, or a remaining part of multi-part substance, such as, for example, a swelling and/or a hardening or cross-linking compound. In response to the filling, bag 220 can fill the gutter and contact the medical devices and/or body lumen defining the gutter substantially reducing the flow of blood and/or bodily fluids through the gutter or allowing removal in whole or in part of plaque or thrombus.

In another example, filler 210 comprises beads. During insertion, bag 220 can be filled with beads or can be empty. Where bag 220 is filled with beads, it can be compressed by a delivery sheath and deploy endoluminally through a medical device delivery system on a catheter to a treatment region. Bag 220 can be positioned in the gutter, vasculature, or medical device. The delivery sheath can be removed or retracted to allow bag 220 to expand and fill the gutter. Alternatively, bag 220 can operatively couple to a lumen of a hollow catheter, in which case the beads can be fed through the catheter lumen to fill bag 220. As bag 220 fills, leakage system or plaque/thrombus reduction device 200 obstructs the gutter and reduces the leakage of blood and/or bodily fluids around the medical devices at the treatment region or contacts the vasculature for removal of portions of plaque or thrombus that are present at the treatment site.

With reference to Figure 4, a leakage reduction system or plaque/thrombus reduction device 300 can comprise an inflatable member 320 and a deployment mechanism 330. Inflatable member 320 can be a balloon, a bladder, a bag or any other suitable structure that is configured to inflate under hydraulic or pneumatic pressure. Inflatable member can comprise texture 310. Texture 310 can be formed on the surface of inflatable member 320. Texture 310 can also be a structural component installed within inflation chamber defined by inflatable member 320 and protrude through the walls of inflatable member 320. Texture 310 can be configured to increase the friction exerted on a surface, when the surface is contacted by inflatable member 320.

Inflatable member 320 can deploy endoluminally through a medical device delivery system in a compressed or deflated configuration. Inflatable member 320 has an inflation cavity operatively couple to a catheter, such that the inflation cavity is in fluid communication with a lumen of a catheter. Upon reaching the treatment site, inflatable member 320 can be positioned in the gutter or medical device or vasculature. In the deflated configuration, inflatable member 320 can receive fluid, such as, for example, water, saline, contrast agent, or other suitable fluid, through the catheter lumen. This fluid causes inflatable member 320 to expand outwardly from the deflated configuration. The expansion can cause inflatable member 320 and associated texture 310 to fill the gutter and engage the medical devices and/or vascular wall defining the gutter or area or fill the area for plaque/thrombus removal. Resulting friction created by the engagement of texture 310 can further retain and stabilize inflatable member 320 in the gutter or allow removal of plaque/thrombus.

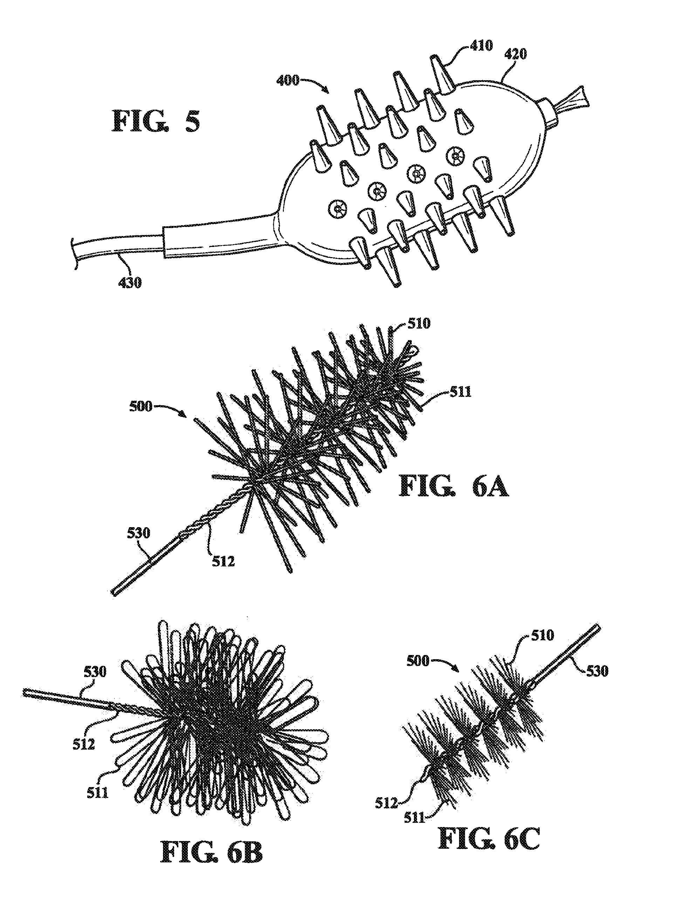

With reference to Figure 5, a leakage reduction system or plaque/thrombus reduction device 400 can comprise a brush 410, an inflatable member 420 and a deployment mechanism 430. Brush 410 can comprise a support column coupled to a plurality of spaced bristles. Inflatable member 420 can be a balloon, a bladder, a bag or any other suitable structure configured to inflate under hydraulic or pneumatic pressure. Brush 410 can be installed within an inflation chamber defined by inflatable member 420, such that the bristles of brush 410 are covered by and elevate portions of the wall of inflatable member 420. Brush 410 can be configured to increase the friction exerted on a surface, when the surface contacts the elevated portions of inflatable member 420.

Inflatable member 420 can deploy endoluminally through a medical device delivery system in a compressed or deflated configuration. The bristles of brush 410 can be sufficiently soft and/or flexible that inflatable member 420 compresses the bristles during deployment through the catheter. The assembly of brush 410 and inflatable member 420 can also be compressed during deployment by a removable delivery sheath. Inflatable member 420 has an inflation cavity and can operatively couple to a catheter, such that the inflation cavity is in fluid communication with a lumen of a catheter. Upon reaching the treatment site, the assembly of brush 410 and inflatable member 420 can be positioned in the gutter or medical device or vasculature for plaque or thrombus removal. In the deflated configuration, inflatable member 420 can receive fluid, such as, for example, water, saline, contrast agent, gel, or other suitable fluid, through the catheter lumen. This fluid causes inflatable member 420 to expand from the deflated configuration. The expansion can cause inflatable member 420 and associated brush 410 to fill the gutter and engage the medical devices and/or vascular wall defining the gutter or engage the medical device or vasculature. Resulting friction created by the engagement of the bristles of brush 410 on the medical devices and/or vascular wall can further retain and stabilize inflatable member 420 in the gutter. The assembly of brush 410 and inflatable member 420 can also be releasably restrained, delivered endoluminally toward the treatment site, and then subsequently unrestrained at or near the treatment site and allowed to expand to fill the gutter where the bristles are sufficient stiff to expand inflatable member 420. Inflatable member 420 can also be filled with fluid through catheter lumen to further stabilize brush 410-inflatable member 420 assembly. The bristles of brush 410 can have openings at their distal ends for drug delivery, in which case a drug or drug mixture can be used to inflate member 420.

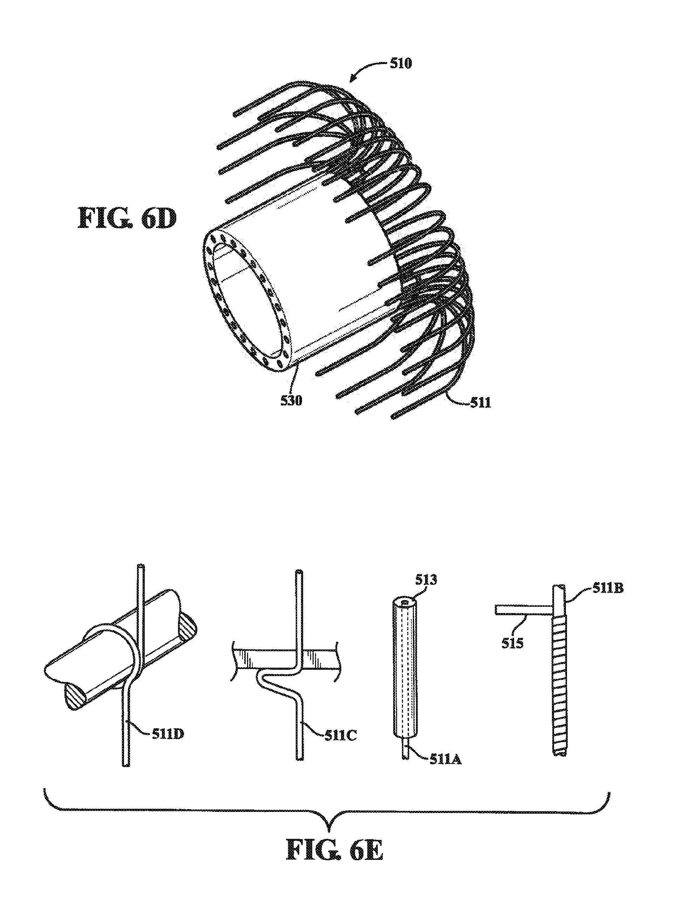

With reference to Figures 6A-6E, a leakage reduction system or plaque thrombus reduction device 500 comprises a brush 510 and a deployment mechanism 530. Brush 510 can comprise a support column 512 and a plurality of bristles 511. Support column 512 can define and/or couple to deployment mechanism 530. Bristles 511 can couple to support column 512 in any suitable orientation. For example, bristles 511 can be distributed along support column 512 in a helical orientation, in disk orientations along one or more spaced diameters of support column 512, in linear orientations along the longitudinal access of support column 512, in a uniform orientation, in a random orientation, or in any other suitable orientation. Bristles 511 can also couple to deployment mechanism 530, such that support column 512 of brush 510 is not required.

Bristles 511 can be of any suitable shape. For example, bristles 511 can be substantially straight, curve at an end, curve over substantially the entire length of bristle 511, and/or the like. Bristles 511 can be made of any suitable material, including, for example, ePTFE, metal, other types of plastics, alloys, composites, or any other suitable material. Bristles 511 can have any suitable rigidity and strength. In one embodiment, e.g., where bristles 511 are substantially soft, brush 510 employs atraumatic anchoring by increasing the number of contact points and decreasing the contact pressure. In other words, brush 510 can comprise a greater number of bristles 511 to increase the contact force exerted on a surface where brush 510 is installed and/or the surface area of the leakage reduction system or plaque/thrombus reduction device. In another embodiment, e.g., where bristles 511 are substantially rigid and strong, brush 510 can have fewer bristles 511.

Bristles 511 can couple to support column 512. In one example, bristle 511 comprises a first end and a second end. The first end of bristle 511 can couple bristle 511 to support column 512 in a cantilevered configuration. In this example, the second end of bristles 511 protrudes outward from the column in a substantially straight or curved configuration. In another embodiment, bristles 511 comprise first and second ends. In this example, both the first end and the second end of bristle 511 attach to support column 512. As such, a curved portion of the length of bristle 511 protrudes from support column 512.

With reference to Figure 6E, bristle 511 can be configured with enhancements. In one embodiment, bristle 511 A can be configured to swell. For instance, bristle 511 A can be covered with a hydrogel. Where bristles 511 A are installed to obstruct fluid flow in a gutter, the bristles can swell to provide more effective occlusion of the gutter. In other examples, bristle 511 B can be wrapped in a fluoropolymer such as ePTFE. The wrap can provide, strength, durability, wear resistance, insertability, biocompatibility, and increased surface area, among other advantages. In these examples, the fluoropolymer can protect and prevent bristles 511 B from becoming damaged or contaminated prior to and during deployment to a treatment region. Similarly, the fluoropolymer can protect the surrounding vasculature. The fluoropolymer coating can also act as a lubricant, for example, when brush 510 is installed within a gutter, such that bristles contact at least one of a medical device and a vascular wall. The fluoropolymer coating may also be configured with an engineered microstructure which could facilitate cellular ingrowth, which assists in mitigation of migration. Similarly, porous microstructure can be an ideal place to imbibe a particular therapeutic agent.

In another example, bristles 511C can include a depth stop. The depth stop can be configured to selectively set the length of bristle that penetrates a vessel wall or protrudes from a brush and support column. In yet another example, bristles 511 can be configured with "S" bends. The "S" bends can limit how far the bristles protrude from the support column, allowing the size and shape of leakage reduction system or plaque/thrombus reduction device 500 to be adjustable. They can also limit how far bristles penetrate surrounding tissues or other endovascular devices. In various examples, bristle 511 D can be formed or wrapped about the support column or guidewire which can provide a guidewire pathway. When installed in a gutter, wrapped bristles can provide a brush with greater surface area to facilitate tissue in-growth and coagulation and/or to provide an increased surface area. Moreover, these enhancements can increase, the effectiveness and life of leakage reduction system or plaque/thrombus reduction device 500.

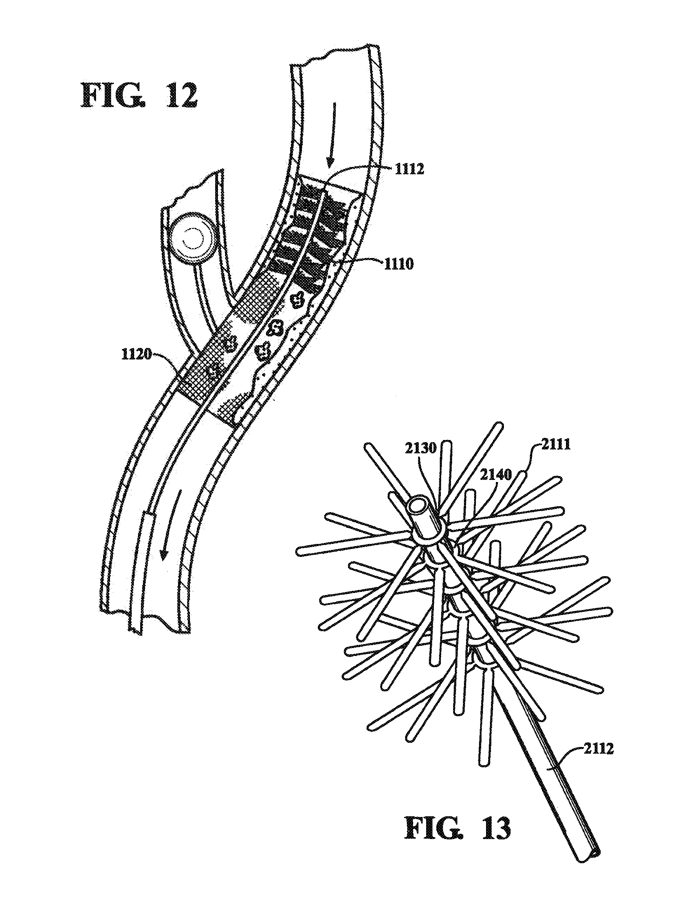

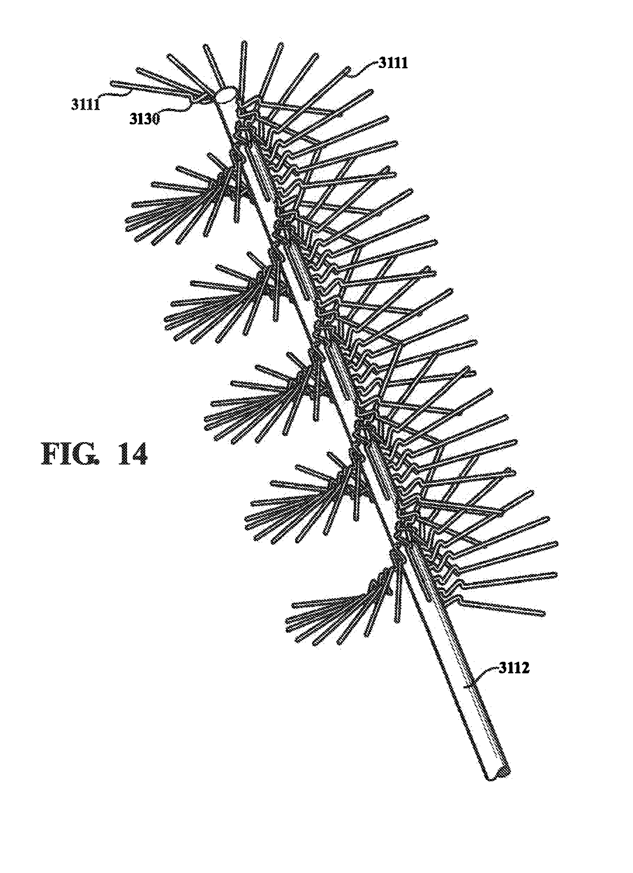

In some embodiments, for example, with reference to Figure 13 and Figure 14, bristles 2111 or 3111 can have a bending portion 2130 or 3130 along the length thereof facilitating bending of the bristles between a compressed delivery configuration and an expanded use configuration. As shown in figure 13, the bristles 2111 can extend outwardly from a generally annular collar 2140 fixed securely to the support 2112. The bending portion 2130 can be defined by a portion of the bristles 2111 having a reduced cross section. The bending portion 2130 can be adjacent the collar 2140 or more distal from the collar along the length of the bristle 2111. Alternatively, as shown in figure 14, the bending portion 3130 can be defined by a bend along a portion of the length of the bristles 3111. The bend may have a generally sinusoidal shape, an "S" shape, a zig-zag shape or any other bend shape to facilitate bending of the bristles between a delivery configuration in which the bristles 3111 are compressed against the support 3112 and an expanded use configuration. The bending portion can also be defined by a change in durometer or flex modulus on a portion of the bristle length. The bending portion can also be defined as a strain relief portion included along the length of the bristle. Strain relief can be configured to include a portion that can have a generally sinusoidal shape, an "S" shape, a zig-zag shape or any other bend shape to facilitate bending of the bristles. Strain relief can be configured to include a portion that can have a a portion of the bristles 2111 having a reduced cross section. The strain relief portion can also be defined by a change in durometer or flex modulus on a portion of the bristle length. Stain relief or bending portion can be any structural or configuration or material change that would facilitate bending along a portion of the length of the bristle.

In various embodiments, brush 510 can be configured to connect to a guidewire or catheter of a medical device delivery system. Brush 510 can be collapsible or can be constrained by a sheath as brush 510 deploys endoluminally via the catheter. Upon reaching the treatment site, brush 510 can be installed in the gutter or can be used to remove plaque or thrombus from the treatment site. Bristles 511 can contact the medical devices and/or vascular wall defining the gutter. The contact by bristles 511 can stabilize and retain brush 510 in the gutter. When installed, brush 510 can obstruct the gutter causing more blood and/or bodily fluid to flow through the lumens defined by the medical devices. Depending on the density of bristles 511 on brush 510 some blood and/or bodily fluid can be allowed to leak into or through the gutter.

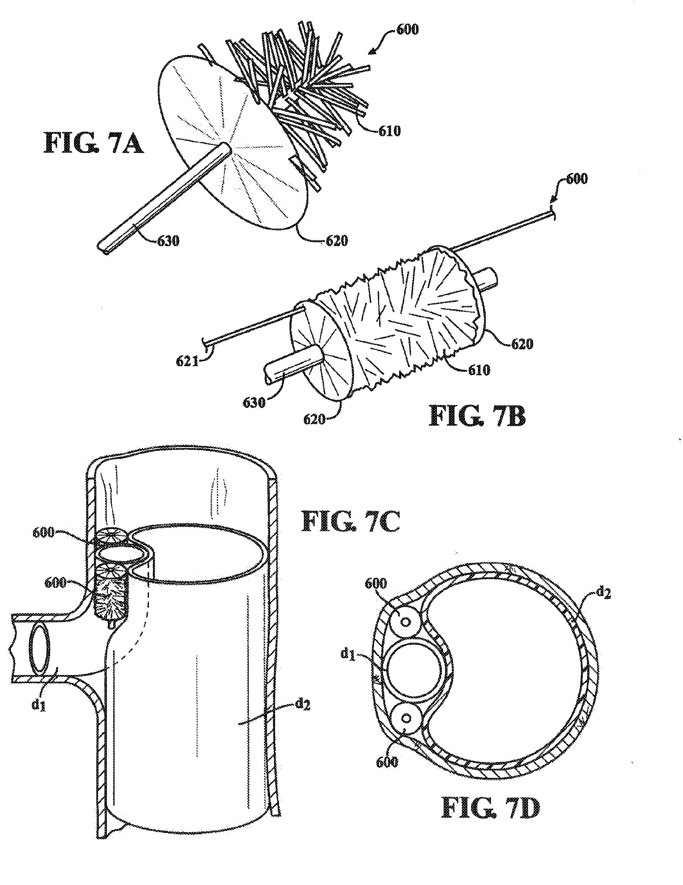

With reference to Figures 7A-7D, a leakage reduction system or plaque/thrombus reduction device 600 comprises a brush 610, an occluder panel 620 and a deployment mechanism 630. Brush 610 can couple to occluder panel 620. Brush 610 can also couple to or define deployment mechanism 630. Moreover, brush 610 can be configured in any suitable fashion as discussed above.

Occluder panel 620 can be any suitable structure to restrict fluid flow. Occluder panel 620 can be configured to operatively couple at any point on the proximal or distal end of brush 610. In various embodiments, one or more occluder panels 620 can couple to each of the proximal end and distal end of brush 610. Occluder panel 620 can couple to one of more bristles or the support column of brush 610 and provide a guidewire pathway for guidewire 621. Occluder panel 620 can laminate a plurality of bristles, which can provide additional strength and rigidity to occluder panel 620. Where occluder panel 620 couples to the support column of brush 610, occluder panel 620 can comprise a frame and/or supports.

In various examples, brush 610 can couple to a guidewire and/or catheter of a medical device delivery system. Brush 610 can be compressed or restrained and deployed through a medical device delivery system to a treatment region. Upon reaching the treatment region, brush 610 can be expanded and installed in the gutter or oppose the intraluminal surface of a deployed stent to remove plaque or thrombus from the treatment region. Brush 610 can be installed in the gutter, such that the bristles of brush 610 contact at least a portion of the medical devices and/or the vascular wall defining the gutter or deployed vascular device, e.g., stent. Occluder panel 620 can further obstruct the gutter. Where brush 610 comprises a first occluder panel 620 at its proximal end and a second occluder panel 620 at its distal end, brush 610 can be placed in the gutter in a releasably restrained configuration. When the brush is released and unrestrained at or near a treatment site, the bristle can expand to stabilize the brush in the gutter and restrict fluid flow through the gutter. Occluder panels 620 can also expand to obstruct fluid flow through the gutter at both the proximal end and distal end of brush 610. Although occluder panels are shown with a generally circular shape, it should be appreciated that the occluder panels may have other alternate shapes or peripheries. The occluder panel on one side of the device may, for example, have a different size or shape than the occluder panel on the other side of the device. Shapes may include roughly triangular, oval, elliptical, trapezoidal, etc.

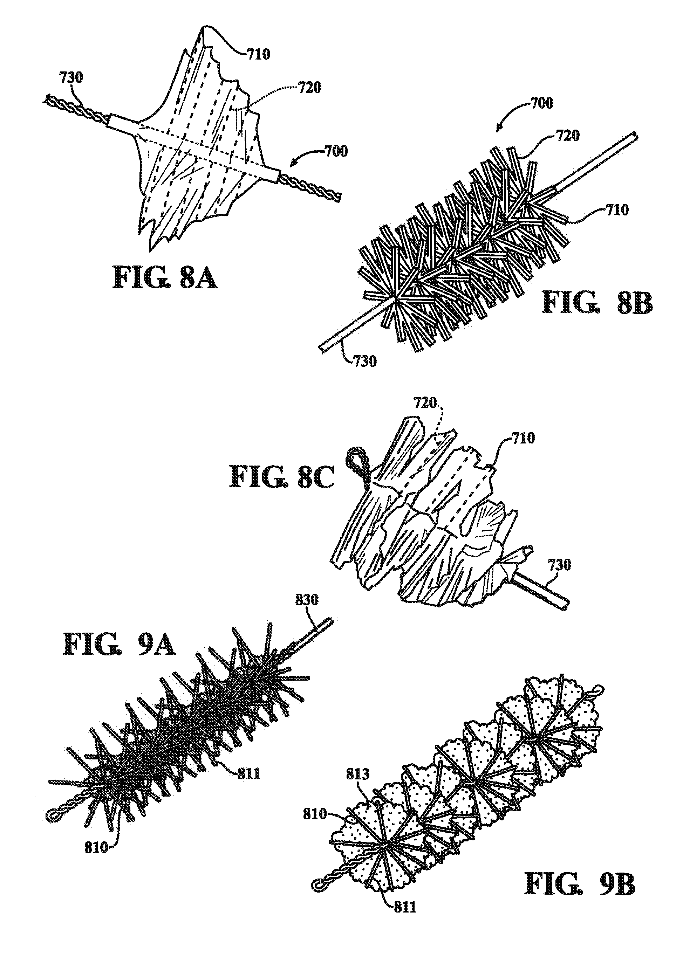

With reference to Figures 8A-8C, a leakage reduction system or plaque/thrombus reduction device 700 can comprise a brush 710, laminated bristles 720, and a deployment mechanism 730. Laminated bristled 720 can be laminated or covered in any suitable fashion. For example, each bristle can be laminated individually, several bristles can be laminated in a single sheet, all of the bristles can be laminated in a helical orientation, all of the bristles can be laminated together, or any other suitable lamination orientation.

Leakage reduction system or plaque/thrombus reduction device 700 can deploy endoluminally through a medical device delivery system to a treatment region in a restrained or compressed configuration. In one example, leakage reduction system or plaque/thrombus reduction device 700 can expand and then be installed in the gutter or used to remove plaque or thrombus from the treatment region. The laminated bristles 720 can contact the medical devices and vascular walls defining the gutter to stabilize leakage reduction system 700. The lamination coupled to the bristles can obstruct the gutter and substantially reduce the leakage through the gutter around the medical devices or atraumatically debride plaque or thrombus protruding through a deployed vascular device. Leakage reduction system or plaque/thrombus reduction device 700 can also be installed in the gutter in a releasably restrained or compressed configuration and then allowed to expand, to reduce the leakage at the gutter.

With reference to Figure 9A-9B, a leakage reduction system or plaque/thrombus reduction device 800 comprises a brush 810, and a deployment mechanism 830. Brush 810 can further comprise a support column and bristles 811. The support column can define a channel having one or more nozzles coupling the channel to an outer surface of the support column. Brush 810 can be configured to receive a substance 813 from a medical device delivery system though the channel defined by the support column. The substance 813 can seep out between the bristles 811. The substance 813 can be any suitable space filler, such as, liquid embolic space filler foam. Upon installation of the brush 810 in the gutter region the substance 813 can seep between the bristles 81 1 to fill the voids between the bristles 811 and substantially reduce the leakage through the gutter.

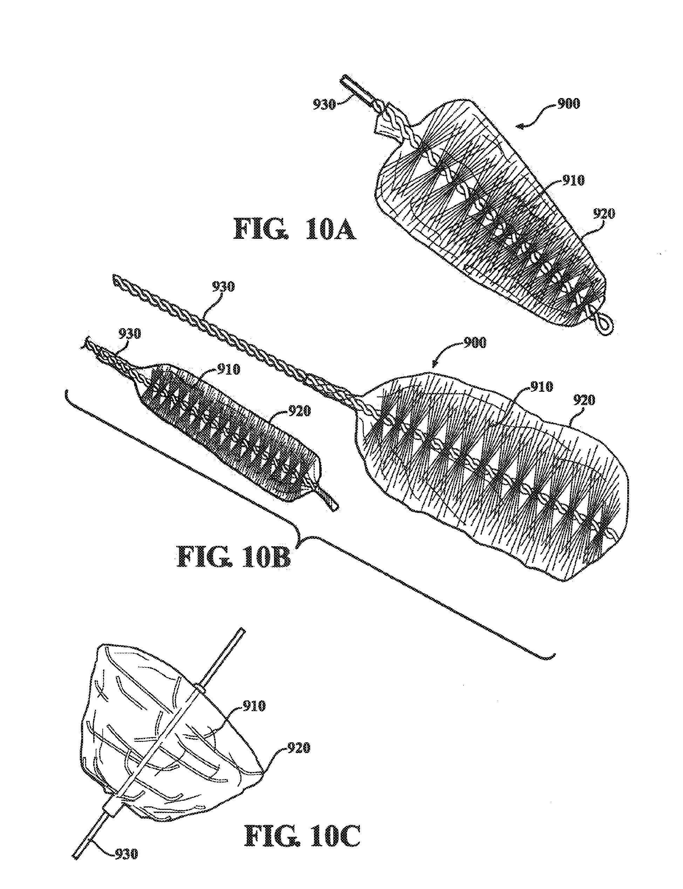

With reference to Figures 10A-10C, leakage reduction system or plaque/thrombus reduction device 900 comprises a brush 910, a bag 920, and a deployment mechanism 930. Brush 910 can install with bag 920 such that bag 920 covers at least a portion of the bristles of brush 910. Bag 920 can be of any suitable size and shape to cover brush 910. In one example, bag 920 is made of a fluoropolymer such as ePTFE. However, bag 920 can be made of any suitable biocompatible material.

In order to achieve proper deployment, bag 920 can be formed from a fluoropolymer having pore sizes between about 50 and 1000pm. Such pore sizes in bag 920 allow for greater fluid flows through the bag, which can allow for a lower force to expand the bag. This can be particularly useful, for example, where the bristles of brush 910 are made of a soft and flexibly material, such as, ePTFE, and may not have sufficient rigidity to expand bag 920 when bag 920 is subjected to a fluid flow. The increased fluid flow can also assist with the deployment of bag 920 by pulling the bag from a compressed configuration to an expanded configuration. Where the larger pores are not sufficient to allow for proper deployment, bag 920 can be hydrophilic. Where bag 920 is hydrophilic, the increased fluid flow through created by the hydrophilic properties can further expand bag 920. In a configuration where bag 920 is perforated, bristles from indwelling brush 910 can extend through perforations and engage the tissue and/or adjacent endovascular devices, such engagement of bristles can minimize migration of device or enhance plaque or thrombus removal.

The assembly of brush 910 and bag 920 can be of any suitable size and shape for substantially reducing the leakage through a gutter. Upon deployment through a medical device delivery system to a treatment region installation of the assembly of brush 910 and bag 920 can be expanded and installed in the gutter/vasculature/medical device or installed within the gutter/vasculature/medical device in a constrained configuration and allowed to expand. If the assembly of brush 910 and bag 920 tends to be a difficult to open once placed in the gutter/vasculature/medical device, the assembly can be expanded prior to installation in the g utter/vasculature/medical device.

The leakage reduction system can be used independently to obstruct flow through a vessel; or to occlude anatomical features such as left atrial appendage; or to obstruct flow though a cardiac defect.

Turning to other examples, a leakage reduction system can comprise a first medical device and a second medical device. First medical device can be any suitable medical device defining a lumen that is installable within the vasculature. The lumen defined in first medical device can have a cross-section that corresponds to at least a portion of the cross-sectional area of the treatment region of the vasculature. Similarly, second medical device can be any suitable medical device defining a lumen that is installable within the vasculature. The lumen defined in second medical device can have a cross-section that corresponds to at least a portion of the cross-sectional area of the treatment region of the vasculature. First medical device and second medical device can have corresponding shapes, such that when installed together in the vasculature, first medical device and second medical device cover substantially all of the cross-sectional area of a treatment region in the vasculature, thus eliminating the presence of the gutters.

The first medical device comprises any device or structure configured to provide and/or support a therapeutic use in a vasculature. In various embodiments, first medical device can be any medical device that can be delivered endovascularly. For example, first medical device can be a stent or stent graft, filter, valves, bifurcated stents, drug-delivering devices, such as drug-eluting stents, oncology therapies, spacers, optical devices, markers and/or other similar devices.

Similarly, an exemplary second medical device comprises any device or structure configured to provide and/or support a therapeutic use in a vasculature. In various embodiments, second medical device can be any medical device that can be delivered endovascularly. For example, second medical device can be a stent or stent graft, filter, valves, bifurcated stents, drug-delivering devices, such as drug- eluting stents, oncology therapies, spacers, optical devices, markers and/or other similar devices.

Each of first medical device and second medical device can be of any suitable size and shape. First medical device can comprise a first surface that conforms to a wall of the vasculature. Similarly, second medical device can comprise a first surface that conforms to a wall of the vasculature. First medical device and second medical device can each comprise a second surface. These second surfaces can have corresponding shapes, such that the surfaces conform with one another with first medical device and second medical device are installed in a treatment region in the vasculature. For example, the second surface of first medical device and the second surface of second medical device can both be flat. The second surface of first medical device and the second surface of second medical device can be convex and concave, respectively, such that the bulge of the concave surface corresponds to the depression of the concave surface. Moreover, the second surface of first medical device and the second surface of second medical device can have any suitable corresponding shapes. In this example, the resulting lumen formed by first medical device and second medical device is substantially similar to the lumen formed by the vasculature. As such, leakage at the treatment site is substantially reduced or eliminated, because blood and/or bodily fluid flowing through the vasculature lumen is conducted through the resulting lumen defined by first medical device and second medical device.

In various examples, first medical device and/or second medical device can be flexible. In various examples, one of first medical device and second medical device can be rigid and one of first medical device and second medical device can be flexible, such that the flexible medical device conforms to the shape of the rigid medical device when the devices are installed in the vasculature.

In various examples, first medical device and second medical device can comprise grafts and/or coatings. The coatings of the device can be in contact, such that the cross-sectional area of first medical device and second medical device more closely approximates the cross-sectional area of the vascular lumen.

In various examples, any of the brushes as described herein and shown in Figures 2-10 can be used to remove plaque or thrombus in the vasculature. The brushes can also be configured to remove plaque from medical devices. For example, a medical device, such as a stent, can define a lumen. When installed in the vasculature plaque can protrude through the frame of the medical device into the device's lumen. The brush can enter the lumen and dislodge the plaque that protrudes into the lumen. The brushes (e.g. one of more of the bristles or the brush itself) can also comprise a marker, such as, for example, a radiopaque marker and/or radiopaque bristle. The brush can be delivered by any suitable medical device delivery system, which can include a catheter and/or guidewire.

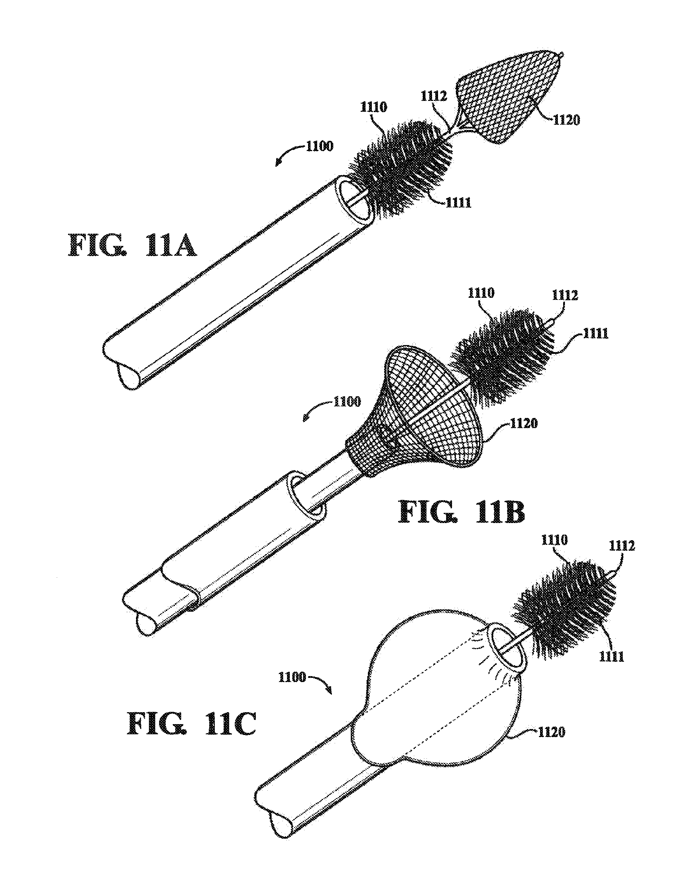

With reference to Figures 11A-C and Figure 12, the brushes can be used or configured with a plaque or emboli capture system, including, for example, a flow reversal system, a suction or aspiration system, a flow stagnation system, a proximal embolic protection device, a distal embolic protection device, a basket, a net, a bucket, or any other suitable embolic capture system or device.

In various examples, a plaque reduction system 1100 comprises a brush 1110. Brush 1110 can be a radiopaque, compliant, catheter-delivered, "bottle brush" plaque reduction device. Brush 1100 can comprise bristles 1111 coupled to a support column 1112. Bristles 1111 can be made of any suitable material, including, for example, nitinol, stainless steel, alloys, nylon, PTFE, composite, or the like. A plaque reduction system 1100 can further comprise an emboli capture system 1120, which can be located in the proximal or distal region of brush 1110. In this regard, it can act as a leading or a trailing filter. Embolic protection device 1120 can comprise a filter sack, such as an ePTFE filter sack, to capture embolic debris.

In operation, brush 1110 can be introduced through a medical device delivery system to a treatment region. After substantial flow stagnation or flow reversal is established, and distal circulation is protected, brush 1110 can be passed through a medical device lumen and can deploy at the distal end of the medical device, such as, for example, a stent. Brush 1110 can be delivered such that bristles 1111 deploy at the distal end of the deployed medical device without exiting the medical device and disrupting the adjacent "normal" vasculature. Brush 1110 can be retracted, advanced, and/or twisted, and done so repeatedly or as necessary, in the lumen of the deployed medical device to remove any atherosclerotic debris, which can protrude through the medical device frame into the lumen. The protruding plaque can be dislodged by brush 1110 and captured by the emboli capture system, such as an embolic protection device. As late embolic events and progression of disease have been associated with plaque protrusion post stent delivery, this system would significantly contribute to the reduction of late catastrophic events and significantly improve the efficacy of the stenting procedure.

In various examples, brush 1110 can be mounted on the tip of a wire, such as a 0.36 mm (.014 inch) or other suitably sized wire, and be delivered via a catheter. Bristles 1111 would not deform the stented vessel. Bristles 1111 can be shaped and oriented in any suitable fashion. In one embodiment, bristles 1111 can be oriented as "stacked V's" or "stacked chevrons," whether facing forward, backward, or otherwise. Such configurations can be relatively easy to deploy and retrieve. In various embodiments, plaque reduction system 1100 can be configured to selectively deploy and/or release brush 1110.

The support structures, coatings and secondary structures, described above, are highly bio-compatible. As used herein, a "biocompatible material" is a material suited for and meeting the purpose and requirements of a medical device, used for either long or short term implants or for non-implantable applications. Long term implants are defined as items implanted for more than 30 days. These support structures, coatings, and secondary structures can be formed of a fluoropolymer, such as ePTFE. Alternatively, or in combination with a fluoropolymer, the support structures, coatings, and secondary structures can be formed of biocompatible materials, such as polymers, which can include fillers such as metals, carbon fibers, Dacron, glass fibers or ceramics. Such polymers can include olefin polymers, polyethylene, polypropylene, polyvinyl chloride, polytetrafluoroethylene which is not expanded, fluorinated ethylene propylene 45 copolymer, polyvinyl acetate, polystyrene, poly(ethylene terephthalate), naphthalene dicarboxylate derivatives, such as polyethylene naphthalate, polybutylene naphthalate, polytrimethylene naphthalate and trimethylenediol naphthalate, polyurethane, polyurea, silicone rubbers, polyamides, polycarbonates, polyaldehydes, natural rubbers, polyester copolymers, styrenebutadiene copolymers, polyethers, such as fully or partially halogenated polyethers, copolymers, and combinations thereof. Also, polyesters, including polyethylene terephthalate (PET) polyesters, polypropylenes, polyethylenes, polyurethanes, polyolefins, polyvinyls, polymethylacetates, polyamides, naphthalane dicarboxylene derivatives, and natural silk can be included in support structures, coatings and secondary structures.

These support structures, coatings and secondary structures can be utilized with bio-active agents. Bio-active agents can be coated onto a portion or the entirety of the support structures, coatings and secondary structures for controlled release of the agents once the support structures, coatings and secondary structures is implanted. The bio-active agents can include, but are not limited to, vaso constrictors, thrombogenic agents such as thrombin. Bio-active agents can also include, for example, vasodilators, anti-coagulants, such as, for example, warfarin and heparin. Other bio-active agents can also include, but are not limited to agents such as, for example, anti-proliferative/antim<'>rtotic agents including natural products such as vinca alkaloids (i.e. vinblastine, vincristine, and vinorelbine), paclitaxel, epidipodophyllotoxins (i.e. etoposide, teniposide), antibiotics (dactinomycin (actinomycin D) daunorubicin, doxorubicin and idarubicin), anthracyclines, mitoxantrone, bleomycins, plicamycin (mithramycin) and mitomycin, enzymes (L- asparaginase which systemically metabolizes L-asparagine and deprives cells which do not have the capacity to synthesize their own asparagine); antiplatelet agents such as G(GP) IIb/IIIa inhibitors and vitronectin receptor antagonists; antiproliferative/antimitotic alkylating agents such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nirtosoureas (carmustine (BCNU) and analogs, streptozocin), trazenes-dacarbazinine (DTIC); anti-proliferative/antimitotic antimetabolites such as folic acid analogs (methotrexate), pyrimidine analogs (fluorouracil, floxuridine, and cytarabine), purine analogs and related inhibitors (mercaptopurine, thioguanine, pentostatin and 2-chlorodeoxyadenosine {cladribine}); platinum coordination complexes (cisplatin, carboplatin), procarbazine, hydroxyurea, mitotane, aminoglutethimide; hormones (i.e. estrogen); anti-coagulants (heparin, synthetic heparin salts and other inhibitors of thrombin); fibrinolytic agents (such as tissue plasminogen activator, streptokinase and urokinase), aspirin, dipyridamole, ticlopidine, clopidogrel, abciximab; antimigratory; antisecretory (breveldin); antiinflammatory: such as adrenocortical steroids (Cortisol, cortisone, fludrocortisone, prednisone, prednisolone, 6a-methylprednisolone, triamcinolone, betamethasone, and dexamethasone), non-steroidal agents (salicylic acid derivatives i.e. aspirin; para-aminophenol derivatives i.e. acetaminophen; indole and indene acetic acids (indomethacin, sulindac, and etodalac), heteroaryl acetic acids (tolmetin, diclofenac, and ketorolac), arylpropionic acids (ibuprofen and derivatives), anthranilic acids (mefenamic acid, and meclofenamic acid), enolic acids (piroxicam, tenoxicam, phenylbutazone, and oxyphenthatrazone), nabumetone, gold compounds (auranofin, aurothioglucose, gold sodium thiomalate); immunosuppressives: (cyclosporine, tacrolimus (FK-506), sirolimus (rapamycin), azathioprine, mycophenolate mofetil); angiogenic agents: vascular endothelial growth factor (VEGF), fibroblast growth factor (FGF); angiotensin receptor blockers; nitric oxide donors; anti-sense oligionucleotides and combinations thereof; cell cycle inhibitors, mTOR inhibitors, and growth factor receptor signal transduction kinase inhibitors; retenoids; cyclin/CDK inhibitors; HMG co-enzyme reductase inhibitors (statins); and protease inhibitors.

As used herein, the term "bio-resorbable" includes a suitable biocompatible material, mixture of materials or partial components of materials being degraded into other generally non-toxic materials by an agent present in biological tissue (i.e., being bio-degradable via a suitable mechanism, such as, for example, hydrolysis) or being removed by cellular activity (i.e., bioresorption, bioabsorption, or bioresorbable), by bulk or surface degradation (i.e., bioerosion such as, for example, by utilizing a water insoluble polymer that is soluble in water upon contact with biological tissue or fluid), or a combination of one or more of the bio-degradable, bio- erodable, or bio-resorbable material noted above. Potential materials for the stent described herein include, for example, biodegradable polymers such as polylactic acid, i.e., PLA, polyglycolic acid, i.e., PGA, polydioxanone, i.e., PDS, polyhydroxybutyrate, i.e., PHB, polyhydroxyvalerate, i.e., PHV and copolymers or a combination of PHB and PHV (available commercially as Biopol(R), polycaprolactone (available as Capronor(R)), polyanhydrides (aliphatic polyanhydrides in the back bone or side chains or aromatic polyanhydrides with benzene in the side chain), polyorthoesters, polyaminoacids (e.g., poly-L-lysine, polyglutamic acid), pseudo-polyaminoacids (e.g., with back bone of polyaminoacids altered), polycyanocrylates, or polyphosphazenes; as well as bioresorbable metals or metal alloys.

Thus, the leakage reduction system described herein provides a mechanism to obstruct and substantially reduce the leakage through a gutter. The plaque reduction system described herein provides a mechanism to remove plaque or thrombus from the vasculature and or medical device.

Finally, the present disclosure has been described above with reference to various examples. It should be appreciated that the various examples shown and described herein are illustrative of the present disclosure and its best mode and are not intended to limit in any way the scope of the present disclosure. Those skilled in the art having read this disclosure will recognize that changes and modifications can be made to the various examples without departing from the scope of the present disclosure. For example, various aspects and embodiments of the present disclosure can be used to provide other methods of treatment, such as drug eluting stents, and/or for imaging purposes. Although certain preferred aspects of the present disclosure are described herein in terms of embodiments, such aspects of the present disclosure can be achieved through any number of suitable means now known or hereafter devised. Accordingly, these and other changes or modifications are intended to be included within the scope of the present disclosure.

## Claims

1. A medical device including a plaque reduction brush said brush comprising:
at least one support (2112) adapted to allow said brush to be endoluminally deliverable via a delivery device to a treatment site; and
a plurality of bristles (2111) extending from the at least one support and being arranged in a predetermined pattern along the at least one support, each of the plurality of bristles having a length, wherein one or more of the plurality of bristles has a bending portion (2130) along the length that is more easily bent than another portion of the length thereof, said bending portion facilitating bending of the one or more of the plurality of bristles between a compressed delivery configuration and an expanded use configuration.

2. The medical device of claim 1, wherein the delivery device is a guidewire.

3. The medical device of claim 1, further comprising a secondary structure.

4. The medical device of claim 3, wherein the secondary structure is a bag (920).

5. The medical device of claim 4, wherein said bag (920) substantially covers one or more of the plurality of bristles (2111).

6. The medical device of claim 5, wherein said bristles at least partially protrude from the bag.

7. The medical device of claim 6, wherein said bag (920) comprises a fluoropolymer.

8. The medical device of claim 1, wherein the one or more of the plurality (2111) of bristles is formed from nitinol.

9. The medical device of claim 1, wherein the plurality of bristles (2111) is polymer, optionally
wherein the one or more of the plurality of bristles has a substantially uniform durometer along the length thereof, the bending portion (2130) having a reduced durometer.

10. A medical device of claim 1, wherein the plurality of bristles (2111) incorporate a strain relief adjacent to the support, or
wherein the plurality of bristles are laminated in a fluoropolymer.

11. The medical device of claim 1, wherein the one or more of the plurality of bristles (2111) has a substantially uniform cross section along the length thereof, the bending portion being defined by a portion of the one or more of the plurality of bristles having a reduced cross section, optionally
wherein the one or more of the plurality of bristles extends outwardly from a generally annular collar (2140) fixedly secured to the support, the reduced width portion being adjacent the collar, and further optionally
wherein the one or more of the plurality of bristles has a generally flattened cross section.

12. The medical device of claim 1, wherein the bending portion (2130) is defined by a generally sinusoidal-shaped bend along a portion of the length of the one or more of the plurality of bristles (2111), or
wherein the bending portion is defined by a change of flex modulus along the length of the bristle.

13. The medical device of claim 1, wherein the predetermined path from which the plurality of bristles (2111) protrude from the support extends generally helically and obliquely about the support.

14. The medical device of claim 1, wherein the one or more of plurality of bristles (2111) is covered, or
wherein the one or more of plurality of bristles is covered where in the covering is a hydrogel, or
wherein the one or more of plurality of bristles is covered in a fluoropolymer.

15. The medical device of claim 1, wherein the one or more of plurality of bristles (2111) is covered where in the covering is a ptfe tape wrapping, optionally
wherein the tape wrapping has a varying slit width, or optionally
wherein the tape wrapping has a varying wrap angle.

## Patentansprüche

1. Medizinische Vorrichtung, umfassend eine Plaquereduktionsbürste, wobei die Bürste Folgendes umfasst:
mindestens eine Stütze (2112), die dafür ausgelegt ist, zu ermöglichen, dass die Bürste endoluminal über eine Zuführvorrichtung einer Behandlungsstelle zugeführt werden kann; und
eine Mehrzahl von Borsten (2111), die sich aus der mindestens einen Stütze erstreckt und in einem vorgegebenen Muster entlang der mindestens einen Stütze angeordnet ist, wobei jede der Mehrzahl von Borsten eine Länge aufweist, wobei eine oder mehrere der Mehrzahl von Borsten einen Biegeabschnitt (2130) entlang der Länge aufweisen, der sich leichter biegen lässt als ein anderer Abschnitt der Länge davon, wobei der Biegeabschnitt das Biegen der einen oder mehreren der Mehrzahl von Borsten zwischen einer komprimierten Zuführkonfiguration und einer ausgedehnten Gebrauchskonfiguration erleichtert.

2. Medizinische Vorrichtung nach Anspruch 1, wobei die Zuführvorrichtung ein Führungsdraht ist.

3. Medizinische Vorrichtung nach Anspruch 1, ferner umfassend eine Sekundärstruktur.

4. Medizinische Vorrichtung nach Anspruch 3, wobei die Sekundärstruktur ein Beutel (920) ist.

5. Medizinische Vorrichtung nach Anspruch 4, wobei der Beutel (920) im Wesentlichen eine oder mehrere der Mehrzahl von Borsten (2111) abdeckt.

6. Medizinische Vorrichtung nach Anspruch 5, wobei die Borsten zumindest teilweise aus dem Beutel hervorragen.

7. Medizinische Vorrichtung nach Anspruch 6, wobei der Beutel (920) ein Fluorpolymer umfasst.

8. Medizinische Vorrichtung nach Anspruch 1, wobei die eine oder mehreren der Mehrzahl von Borsten (2111) aus Nitinol geformt sind.

9. Medizinische Vorrichtung nach Anspruch 1, wobei die Mehrzahl von Borsten (2111) ein Polymer ist, gegebenenfalls
wobei die eine oder mehreren der Mehrzahl von Borsten ein im Wesentlichen einheitliches Durometer entlang der Länge davon aufweisen, wobei der Biegeabschnitt (2130) ein verringertes Durometer aufweist.

10. Medizinische Vorrichtung nach Anspruch 1, wobei die Mehrzahl von Borsten (2111) eine Zugentlastung umfasst, die an die Stütze angrenzt, oder
wobei die Mehrzahl von Borsten mit einem Fluorpolymer laminiert ist.

11. Medizinische Vorrichtung nach Anspruch 1, wobei die eine oder mehreren der Mehrzahl von Borsten (2111) entlang der Länge davon einen im Wesentlichen einheitlichen Querschnitt aufweisen, wobei der Biegeabschnitt durch einen Abschnitt der einen oder mehreren der Mehrzahl von Borsten definiert ist, der einen verringerten Querschnitt aufweist, gegebenenfalls
wobei sich die eine oder mehreren der Mehrzahl von Borsten von einem im Allgemeinen ringförmigen Bund (2140) nach außen erstrecken, der fest an der Stütze befestigt ist, wobei der Abschnitt mit verringerter Breite an den Bund angrenzt und ferner gegebenenfalls
wobei die eine oder mehreren der Mehrzahl von Borsten einen im Allgemeinen abgeflachten Querschnitt aufweisen.

12. Medizinische Vorrichtung nach Anspruch 1, wobei der Biegeabschnitt (2130) durch eine im Allgemeinen sinusförmige Biegung entlang eines Abschnitts der Länge der einen oder mehreren der Mehrzahl von Borsten (2111) definiert ist oder
wobei der Biegeabschnitt durch eine Veränderung des Biegemoduls entlang der Länge der Borste definiert ist.

13. Medizinische Vorrichtung nach Anspruch 1, wobei sich der vorgegebene Pfad, über den die Mehrzahl von Borsten (2111) aus der Stütze hervorragt, im Allgemeinen spiralförmig und schräg um die Stütze erstreckt.

14. Medizinische Vorrichtung nach Anspruch 1, wobei die eine oder mehreren der Mehrzahl von Borsten (2111) abgedeckt sind oder
wobei die eine oder mehreren der Mehrzahl von Borsten abgedeckt sind, wobei die Abdeckung ein Hydrogel ist oder
wobei die eine oder mehreren der Mehrzahl von Borsten von einem Fluorpolymer abgedeckt sind.

15. Medizinische Vorrichtung nach Anspruch 1, wobei die eine oder mehreren der Mehrzahl von Borsten (2111) abgedeckt sind, wobei die Abdeckung eine Umhüllung aus einem PTFE-Band ist, gegebenenfalls
wobei die Bandumhüllung eine variierende Schlitzbreite aufweist oder gegebenenfalls
wobei die Bandumhüllung einen variierenden Umhüllungswinkel aufweist.

## Revendications

1. Dispositif médical incluant une brosse de réduction de plaque, ladite brosse comprenant :
au moins un support (2112) conçu pour permettre à la brosse d'être posée par voie endoluminale par un dispositif de pose au niveau d'un site de traitement ; et
une pluralité de poils (2111) s'étendant à partir dudit au moins un support et étant agencés selon un motif prédéfini le long dudit au moins un support, chacun parmi la pluralité de poils ayant une longueur, un ou plusieurs poils parmi la pluralité de poils ayant une partie de courbure (2130) le long de la longueur qui est plus facilement courbée qu'une autre partie de la longueur de ceux-ci, ladite partie de courbure facilitant la courbure desdits un ou plusieurs poils parmi la pluralité de poils entre une configuration de pose comprimée et une configuration d'utilisation déployée.

2. Dispositif médical selon la revendication 1, ledit dispositif de pose étant un fil-guide.

3. Dispositif médical selon la revendication 1 comprenant en outre une structure secondaire.

4. Dispositif médical selon la revendication 3, ladite structure secondaire étant une poche (920).

5. Dispositif médical selon la revendication 4, ladite poche (920) recouvrant pratiquement un ou plusieurs poils parmi la pluralité de poils (2111).

6. Dispositif médical selon la revendication 5, lesdits poils dépassant au moins en partie de la poche.

7. Dispositif médical selon la revendication 6, ladite poche (920) comprenant un fluoropolymère.

8. Dispositif médical selon la revendication 1, lesdits un ou plusieurs poils parmi la pluralité de poils (2111) étant constitués de nitinol.

9. Dispositif médical selon la revendication 1, ladite pluralité de poils (2111) étant un polymère, éventuellement
lesdits un ou plusieurs poils parmi la pluralité de poils ayant une dureté mesurée par duromètre pratiquement uniforme le long de la longueur de ceux-ci, la partie de courbure (2130) ayant une dureté mesurée par duromètre réduite.

10. Dispositif médical selon la revendication 1, ladite pluralité de poils (2111) incorporant un dispositif de relâchement de contraintes adjacent au support, ou
ladite pluralité de poils étant stratifiée dans un fluoropolymère.

11. Dispositif médical selon la revendication 1, lesdits un ou plusieurs poils parmi la pluralité de poils (2111) ayant une section transversale pratiquement uniforme le long de la longueur de ceux-ci, la partie de courbure étant définie par une partie desdits un ou plusieurs poils parmi la pluralité de poils ayant une section transversale réduite, éventuellement
lesdits un ou plusieurs poils parmi la pluralité de poils s'étendant vers l'extérieur depuis un collier (2140) généralement annulaire fixé à demeure au support, la partie de largeur réduite étant adjacente au collier, et en outre éventuellement
lesdits un ou plusieurs poils parmi la pluralité de poils ayant une section transversale généralement aplatie.

12. Dispositif médical selon la revendication 1, ladite partie de courbure (2130) étant définie par un coude généralement de forme sinusoïdale le long d'une partie de la longueur desdits un ou plusieurs poils parmi la pluralité de poils (2111), ou
ladite partie de courbure étant définie par un changement de module de flexion le long de la longueur du poil.

13. Dispositif médical selon la revendication 1, ledit chemin prédéfini duquel la pluralité de poils (2111) dépassent du support s'étendant généralement de manière hélicoïdale ou oblique autour du support.

14. Dispositif médical selon la revendication 1, lesdits un ou plusieurs poils parmi la pluralité de poils (2111) étant recouverts, ou
lesdits un ou plusieurs poils parmi la pluralité de poils étant recouverts lorsqu'un hydrogel se trouve dans le revêtement, ou
lesdits un ou plusieurs poils parmi la pluralité de poils étant recouverts dans un fluoropolymère.

15. Dispositif médical selon la revendication 1, lesdits un ou plusieurs poils parmi la pluralité de poils (2111) étant recouverts lorsqu'un ruban d'enrobage en PTFE se trouve dans le revêtement, éventuellement
ledit ruban d'enrobage ayant une largeur de fente variable, ou éventuellement
ledit ruban d'enrobage ayant un angle d'enroulement variable.
